# EUROPEAN PATENT APPLICATION

(11) **EP 4 597 237 A1**
(43) Date of publication of application: **06.08.2025**
(21) Application number: 24305186.9
(22) Date of filing: 02.02.2024
(51) Int. Cl.: G05B 17/02, G06Q 10/04

(54) **GENERATION OF CUSTOMIZED SOFTWARE BASED MODELS FOR PRODUCTION PROCESS APPLICATIONS**

(71) Applicant: Merck Patent GmbH, 64293 Darmstadt (DE)
(72) Inventor: Voltz, Karine Elise, 67120 Molsheim (FR); Eberhard, Philippe, 67120 Molsheim (FR); Gerling, Jean-Luc, 67120 Molsheim (FR); Rebai, Adlane, 67120 Molsheim (FR); Cointe, Nicolas, 92200 Neuilly sur Seine (FR); Hubert, Antoine, 69760 Limonest (FR)
(74) Representative: Merck Patent Association

(57) **Abstract**

Method for establishing and calibrating a customized software-based model for simulating a production process via a software-based Model Builder Module on a computer, wherein the Model Builder Module is connected to an ontology database that contains different domains, such as a bioprocessing data domain, a numeric simulation domain, and a mathematical model domains for the Model Builder Module, comprising the following steps of Defining the goal of the software based model, including information about the process the model describes, in particular process steps, cell line, molecule produced, variables the model predicts and threshold values of required model quality indicators; Selecting experimental data required for the calibration and the validation of the model in form of a list of experiments selectable in the Model Builder Module, wherein the context of the experiment data, which is stored in the ontology database matches the defined goal of the model; Composing the software-based model using the toolset proposed by the Model Builder Module which is specific to the respective process step the model describes; Calibrating the created software-based model with provided experimental data via the Model Builder Module; and Checking the validity and quality of the software-based model by performing a quality check of the Model using different mathematical analysis.

## Description

The hereby described invention discloses a method and a system for establishing and training a customized scientific model for simulating a production process.

### Technical Field

The invention deals with the technological area of model engineering for simulation of experiments in the context of digital twin development for bioprocessing.

### Background and description of the prior art

A digital twin is a virtual representation of a physical system or process that replicates its characteristics, behaviors, and interactions. A model is an essential component of a digital twin as it provides the foundation for simulating and representing the real-world system accurately. By simulating the model, the digital twin can replicate the system's performance, behavior, and response to different inputs and conditions.

Bioprocessing modeling involves the development and application of mathematical models to simulate and predict the behavior and performance of bioprocesses. It encompasses various stages of biomanufacturing, including upstream processes, e.g., cell culture and production or fermentation, downstream processes like purification, filtration and process integration.

Through the application of mathematical modeling and simulation, bioprocessing modeling contributes to the development of efficient, scalable, and cost-effective processes for the production of biopharmaceutical products.

Biological systems exhibit inherent variability due to factors such as genetic differences, cell line heterogeneity, and variations in physiological states. Thus, a model has to be adapted or newly built to account for the potential variances in biological behavior and response between the biological systems. Moreover, each bioprocess has its own set of operating conditions and equipment. Updating model parameters or model structure to handle variability then requires addressing each time a new biological system or a new bioprocess.

However, model engineering is a tedious task that requires specific modeling skills. Not all companies have modeling experts among their resources and therefore each time a new biological system or bioprocesses is studied the help of external consultants is required.

A very general approach of how to build a scientific model engineering workflow has been disclosed by the published work of Daume et al. 2020. This workflow however does not provide any final developed technical solution to solve the task of model building or engineering but just suggests necessary steps which should be addressed by a possible solution. Furthermore executing this workflow still requires significant modeling expertise like knowing which equation can be used, how to set mass balance equation from a reaction scheme, which data can be used for calibration, how to choose initial value for parameters, how to interpret model quality check analysis and so on.

The task of this patent application is therefore to find a way which addresses this problem of allowing any end-users without modeling skills to create models or adapt models for different bioprocesses.

### Summary of the invention

This task has been solved by a Method for establishing and calibrating a customized software-based model for simulating a production process via a software-based Model Builder Module on a computer, wherein the Model Builder Module is connected to an ontology database that contains different domains, such as a bioprocessing data domain, a numeric simulation domain, and a mathematical model domains for the Model Builder Module, comprising the following steps of Defining the goal of the software based model, including information about the process the model describes, in particular process steps, cell line, molecule produced, variables the model predicts and threshold values of required model quality indicators; Selecting experimental data required for the calibration and the validation of the model in form of a list of experiments selectable in the Model Builder Module, wherein the context of the experiment data, which is stored in the ontology database matches the defined goal of the model; Composing the software-based model using the toolset proposed by the Model Builder Module which is specific to the respective process step the model describes; Calibrating the created software-based model with provided experimental data via the Model Builder Module; and Checking the validity and quality of the software-based model by performing a quality check of the Model using different mathematical analysis. The domains provide formal definitions of related concepts organized by topics such as bioprocessing, numeric simulation, and mathematical modeling. Those domains provide therefore structured definitions as a RDF graph, including concepts and relations over those concepts, to depict every single mandatory or optional elements involved in the Digital Twin. This allows for instance to automatically filter within experimental runs only those experiments that contain all the data and measurements required for the model and specified in the "Goal definition" of the module. The model composition page of the Model Builder module also provides the advantage that by using the ontology database, the Digital Twin can have a more structured and standardized knowledge base, which helps in capturing the essential elements of the physical system and its behavior. The ontology database further enables interoperability and information exchange between different digital twins

Advantageous and therefore preferred further developments of this invention emerge from the associated subclaims and from the description and the associated drawings.

One of those preferred further developments of the disclosed method comprise that the software-based model is used as a Digital Twin model that is executed to simulate production bioprocess experiments in a bioreactor by describing the biochemical mechanisms underlying the production bioprocesses occurring in the bioreactor during an experiment. Which model is used for this purpose depends on the respective production bioprocess which shall be evaluated. The application of mathematical modeling and simulation, bioprocessing modeling then contributes to the development of efficient, scalable, and cost-effective processes for the production of biopharmaceutical products.

Another one of those preferred further developments of the disclosed method comprise that after selecting the experiment data, a bioprocess reaction scheme is defined by selecting at least one critical reaction from a list provided by the Model Builder Module. That means that within the software-based Model Builder Module different reaction schemes are available and can be selected by a user, in particular via a respective Graphical User Interface (GUI) provided the Model Builder Module.

Another one of those preferred further developments of the disclosed method comprise that the software-based model is created by defining the critical bioprocess reactions and their kinetics and translating them into a set of equations via the Model Builder Module.

These equations, of which the model bases on, describe the respective behavior and property of the modeled bioprocess including its critical reactions and kinetics. The creation of the equations can be done either manually by a user or by a computer and a software which is fed with the required process information.

Another one of those preferred further developments of the disclosed method comprise that the translation of the critical process reactions into a set of equations is done by implementing an algorithm into the Model Builder Module which automatically generates mass balance equations from a given reactions scheme. If the creation of the equations is done by a computer and a software the preferred way to do so is via an algorithm provided by the Model Builder Module. One type of suitable equations generated by this algorithm are mass balance equations.

Another one of those preferred further developments of the disclosed method comprise that the created and calibrated software-based model is stored in a digital catalogue where it can be selected and used for process enhancements like process optimization, real time predictions or process control. This step should be performed at the end of the modeling creation process for further similar modeling use cases so the user has already a qualified example to work with. If the catalogue is only used internally at the same site or lab or also externally for other sites depends on the modeled bioprocesses and the intentions of the users. The catalogue comprise of the model itself and all its context of creation, like on which experimental runs the model was calibrated, the target and state variables described in the model or the accuracy metrics of the model.

Another one of those preferred further developments of the disclosed method comprise that several mechanistic links are provided for selection, which describe model parameters related to reaction kinetics of the software-based model and are maintained in an equation library in a generic form using the functional unit mock-up (FMU) standard. The mechanistic links in form of formulas from the equation library can be selected by the user via the GUI of the Model Builder Module.

Another one of those preferred further developments of the disclosed method comprise that the Model Builder Module provides a decision assistant function which suggests values for required input parameters, ranges for required parameters initial values and/or required threshold values for metrics, which are based on a rule engine which rules are taken from a digital rule catalog provided by expert knowledge. This helps inexperienced users who might not have the knowledge to provide the required input.

Another one of those preferred further developments of the disclosed method comprise that the quality check is a practical identifiability and/or sensitivity analysis. These quality checks indicate if some model parameters could not be estimated with good enough precision to ensure well determined prediction. The reason is that either these parameters are not meaningful for the prediction or that additional data must be taken into account for the fit.

Another solution of the given task is a method for enhancing a production process by creating a suitable software-based model for the production process according to the method as previously described, simulating the production process on a computer by predicting at least one value for production process parameters with the created software-based model, and enhancing the production process by adapting the process parameters according to the results of the predicted at least one value. Using this model, or Digital Twin, for the production process contributes therefore to the development of efficient, scalable, and cost-effective processes for the production of biopharmaceutical products.

Another one of those preferred further developments of the disclosed methods comprise that a physical model, wherein physical model includes mechanistic models, empirical models, phenomenological models and/or equation based-models, or a machine learning model is used as software-based model. The method and Model Builder Module are mainly designed for use of physical models as described above. It should be understood though, that also machine learning models could be created if the modeled bioprocess and the used hardware, mainly in form of the bioreactor, requires it.

A further solution to the given task is a system for establishing, training and using a customized software-based model and/or enhancing a production process, comprising a computer providing a software based Model Builder Module, an ontology database connected to the computer, input and output means connected to the computer for entering and outputting data by the computer, wherein the system is configured to perform the steps of the previously explained method.

Computer program product and a computer-readable storage medium and/or data carrier signal having stored thereon the computer program product, which comprises instructions which cause the involved computers to perform the method steps of the previously described methods.

### Detailed description of the invention

The method and system according to the invention and functionally advantageous developments of those are described in more detail below with reference to the associated drawings using at least one preferred exemplary embodiment. In the drawings, elements that correspond to one another are provided with the same reference numerals.

The drawings show:
- Figure 1:: a detailed scheme of the implemented steps of the prior art workflow of Daume et al. 2020.
- Figure 2:: a schematic organigramm showing the Model Builder Module in a context of a preferred use case
- Figure 3:: the modeling steps performed by the Merlin model builder module
- Figure 4:: the model composition page of the Model Builder Module
- Figure 5:: an example of a Model verification and publishing process

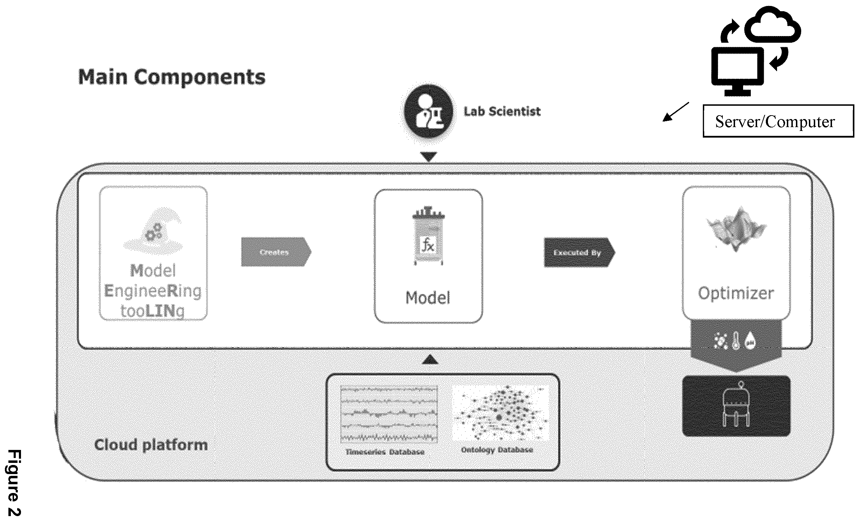

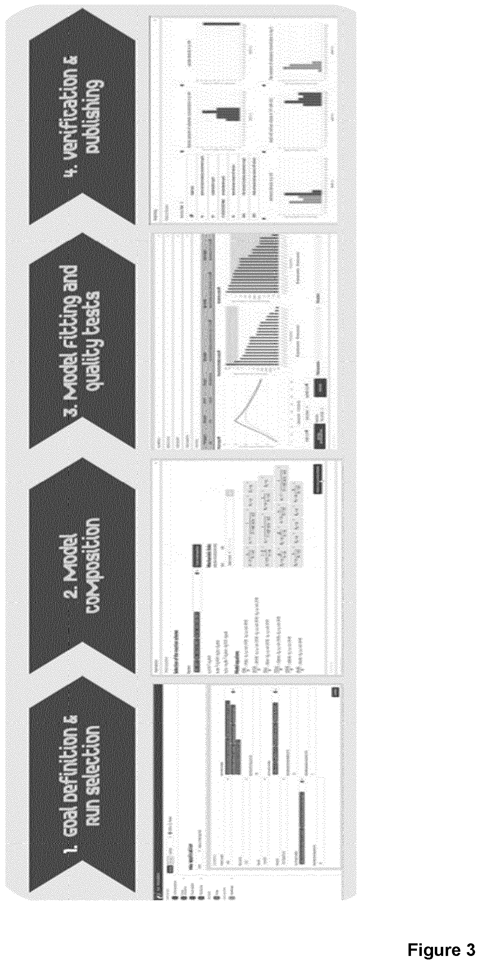

Figure 3 shows an overview about the necessary method steps performed by an application which represents a preferred embodiment. The steps itself are performed divergent in every exemplary embodiment dependent on the different conditions. The system which performs the method including its components in for a specific use case is shown in Figure 2. The shown structure is exemplary for this preferred embodiment. It can change for a other embodiments. Especially the kind of involved computers can differ greatly, depending on how much of the steps is performed by human users with the help of computers and application software or done automatically by specific computers using for instance AI based software.

In the preferred application, a software in form of a model builder module called Merlin is dedicated to the model building task. The kind of models that can be created are varied and depend on the process the model is supposed to simulate. In the peculiar preferred embodiment of the invention described in the following, mechanistic models are used, meaning models made of equations describing the biochemical mechanisms underlying the process occurring in a bioreactor (bioreactions) thus giving a better understanding to the user on this process and enabling him to adapt and enhance the process by applying the gained understanding and insight. These models can be considered as Digital Twins model of the real bioreactor, since they calculate and describe the behavior of the respective bioreactor and its bioreactions.

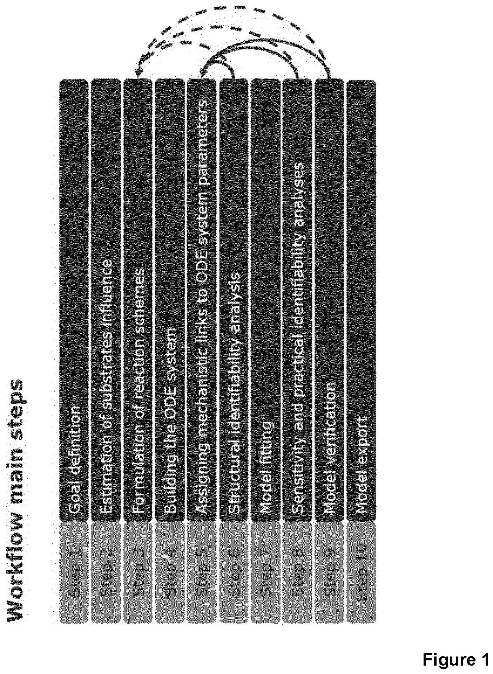

In this module, a workflow including several steps for building a model was programmed and digitalized. The workflow includes the definition of critical reactions and their kinetics, translation into a set of equations of the mathematical model, calibration of the model on available experimental data and checking the validity and quality of the model. This workflow has been adapted from the mentioned published work of Daume et al. 2020 which is shown in Figure 1 which presents a detailed scheme of the implemented steps.

In the Merlin module, steps 1 and 2 of the Daume workflow are addressed in the "Goal definition" step, steps 3 to 6 are part of the "Model composition" step, steps 7 and 8 are covered by the steps "Model fitting and quality tests" and finally model verification and model export to a model catalog is done during the step "Verification and publishing". Figure 3 shows screenshot of the different pages of the Merlin module.

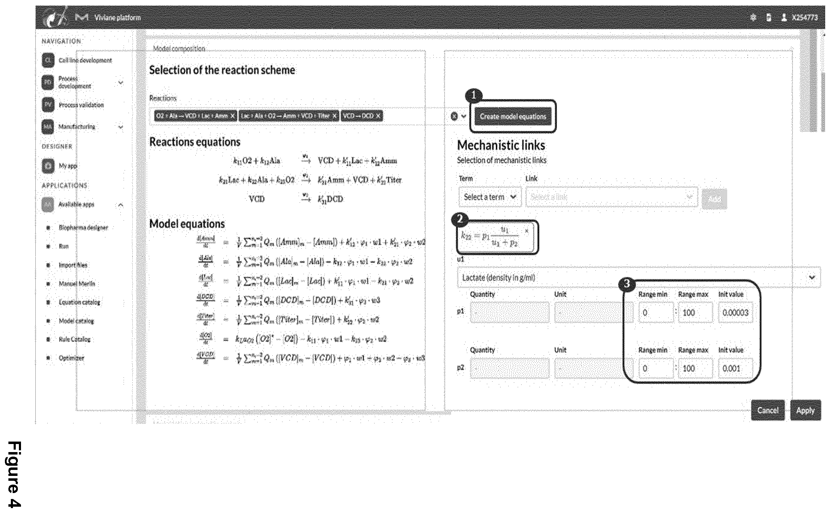

In the following a specific working example shows how the preferred system embodiment, the Merlin module, has adapted the mere scientific and theoretical workflow from the mentioned published work of Daume to be programmed into a real technical application which really allows to create a respective customized model. Figure 4 shows the model composition page of the Merlin module. The indicated squares represent elements from the scientific workflow that were adapted to be programmed in the Merlin module. They consist of three main features:
1) The translation of the reaction schemes into mass balance equations, as shown in Figure 4, where an algorithm has been implemented into the app to automatically generate the mass balance equations from the reactions scheme. This provides the advantage of allowing to create the model in a more efficient manner. The user can directly visualize the correspondence between reactions and model equations
2) An equation library, as seen in Figure 4 which provides several mechanistic links, which are formula for describing parameters of the model, being available to the user selection. They are maintained in an equation library in a generic form using the FMU (functional unit mock-up) standard. Their main advantage is that, depending on the cell line, the clone and other factors, the reaction mechanisms are not the same and that's why the Merlin module provides the user with a wide list of mechanistic links. The user can even add himself mechanistic links via the equation catalog. The Merlin module is very flexible to accommodate customers specific needs.
3) The third feature is a rule-engine disclosed in Figure 4 which provides expertise to an unexperienced user. In the Daume et al. scientific workflow, which is shown in Figure 1, many inputs still have to be fixed and provided by the user. In the Merlin module, values for input parameters, ranges for parameters initial values, threshold values for metrics, are proposed by a "decision assistant" that is based on a rule engine. This engine uses rules from a rule catalog fed by expert knowledge. This assistance based on a rule engine can be extended further in the module to comprise more entries. This will allow non-expert users to use the module by applying the prescribed values of the assistant.

Another feature which is not shown in Figure 4, is an ontology-based data selection. The model builder module Merlin is thereby in a further preferred embodiment connected to an ontology database that contains different domains providing formal definitions of related concepts organized by topics such as bioprocessing, numeric simulation, and mathematical modeling. Merlin is then able to build, play, store and retrieve models as long as they comply with classes, properties and individuals defined in those ontology domains. Those domains provide therefore structured definitions as a RDF graph, including concepts and relations over those concepts, to depict every single mandatory or optional elements involved in the Digital Twin. This allows for instance to automatically filter within experimental runs only those experiments that contain all the data and measurements required for the model and specified in the "Goal definition" of the module, as can be seen in Figure 4. It also allows to publish a model in the model catalog with all its context of creation, e.g. on which experimental runs the model was calibrated, what are the target and state variables described in the model, what are the accuracy metrics of the model and so on. As explained already this provides the advantage that by using the ontology database, the Digital Twin can have a more structured and standardized knowledge base, which helps in capturing the essential elements of the physical system and its behavior. The ontology database further enables interoperability and information exchange between different digital twins.

In the next steps of the results are then displayed on three charts:
First the fitted targets window displays the predicted vs the observed experiments evolution of the titer/viable cell density throughout the time of the experiment for one specific experiment. RMSEp is the root-mean-square-error percentage between the observed and predicted values.
Second the practical identifiability analysis indicates if among the parameters of the model, some could not be estimated with a good enough precision to ensure well determined prediction. On this histogram each bar represents a parameter and the parameters in pink are the ones that could not be fitted with good confidence. The reason is that either these parameters are not meaningful for the prediction or that additional data must be taken into account for the fit.
Third a sensitivity analysis indicates if there are some parameters which are not meaningful for the VCD prediction.

These are the three preferred main kinds of indicators about the quality and consistency of the model. Based on this information, the user can decide to modify the model on the model composition page of module and then to redo the fit and quality check analysis. This can be done in an iterative way.

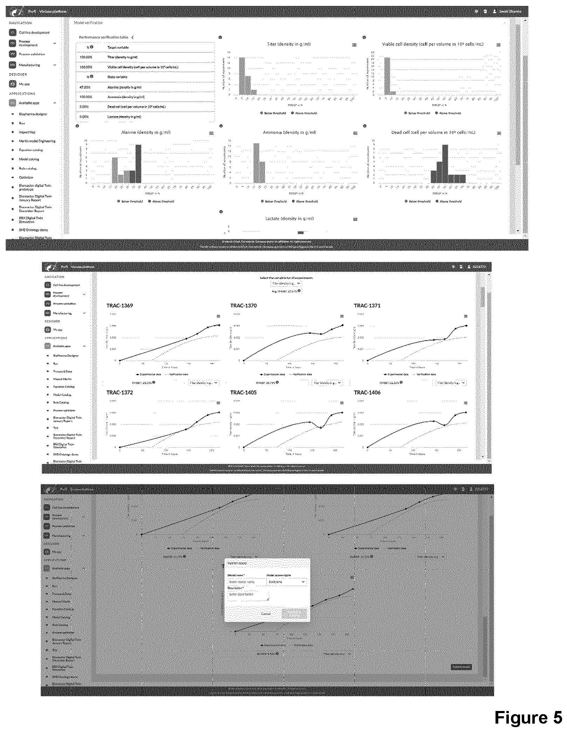

The user can also decide to proceed with the next step, verification. This step of Model verification and publishing is shown exemplary in Figure 5. Figure 5 shows in three different screen shots how the model performs on reproducing the target variable and state variables for experiments that were not used for its calibration. In the performance verification table on screen one it is indicated for each target and state variables predicted by the model, the percentage of runs, e.g. experiments, among the ones selected for verification well reproduced by the model, e.g. for which the error between predicted and observed values is below a defined threshold, as indicated during the previously explained Goal step. Also displayed on Figure 5 is the predicted vs the observed experiments evolution of each target and stage variables for the all individual run selected for the verification, as can be seen in screen two. Finally, the user has the possibility to publish the created model in the model catalog, which is shown by screen three.

The overall advantage of the disclosed invention in form of the preferred embodiment in the working example for the user can be summarized in five points:
1) Codifying knowledge from data and SMEs by implementing mechanistic modelling with minimal code solution.
2) More efficiency in model creation by using the modeling toolset assistance.
3) A model evolves as experiments and associated knowledge grow.
4) Full transparency on the current process knowledge
5) Time saving and centralized source of information.

## Claims

1. Method for establishing and calibrating a customized software-based model for simulating a production process via a software-based Model Builder Module on a computer, wherein the Model Builder Module is connected to an ontology database that contains different domains, such as a bioprocessing data domain, a numeric simulation domain, and a mathematical model domains for the Model Builder Module, the following steps comprising:
• Defining the goal of the software based model, including information about the process the model describes, in particular process steps, cell line, produced molecule, variables the model predicts and threshold values of required model quality indicators;
• Selecting experimental data required for the calibration of the model in form of a list of experiments selectable in the Model Builder Module, wherein the context of the experiment data, which is stored in the ontology database matches the defined goal of the model;
• Composing the software-based model using the toolset proposed by the Model Builder Module which is specific to the respective process step the model describes;
• Calibrating the created software-based model with provided experimental data via the Model Builder Module; and
• Checking the validity and quality of the software-based model by performing a quality check of the Model.

2. Method according to claim 1, wherein
the software-based model is used as a Digital Twin to simulate production bioprocesses in a bioreactor by describing the biochemical mechanisms underlying the production bioprocesses occurring in the bioreactor.

3. Method according to claim 2, wherein
after selecting the experiment data a bioprocess reaction scheme is defined by selecting at least one critical reaction from a list provided by the Model Builder Module.

4. Method according to claim 3, wherein
the software-based model is created by defining the critical bioprocess reactions and translating them into a set of equations via the Model Builder Module.

5. Method according to claim 4, wherein
the translation of the critical process reactions into a set of equations is done by implementing an algorithm into the Model Builder Module which automatically generates mass balance equations from a given reactions scheme.

6. Method according to any of the previous claims, wherein
the created and calibrated software-based model is stored in a digital catalogue where it can be selected and used for process enhancements like process optimization, real time predictions or process control.

7. Method according to any of the previous claims, wherein
several mechanistic links are provided for selection, which describe process parameters of the software-based model and are maintained in an equation library in a generic form using the functional unit mock-up (FMU) standard.

8. Method according to any of the previous claims, wherein
the Model Builder Module provides a decision assistant function which suggests values for required input parameters, ranges for required parameters initial values and/or required threshold values for metrics, which are based on a rule engine taken from a digital rule catalog providing expert knowledge.

9. Method according to any of the previous claims, wherein
the quality check is a practical identifiability and/or sensitivity analysis.

10. Method for enhancing a production process by creating a suitable software-based model for the production process according to the method of claims 1 to 6, simulating the production process on a computer by predicting at least one value for production process parameters with the created software-based model, and enhancing the production process by adapting the process parameters according to the results of the predicted at least one value.

11. Method according to any of the previous claims, wherein
a physical model, wherein physical model includes mechanistic models, empirical models, phenomenological models and/or equation based-models, or a machine learning model is used as software-based model.

12. System for establishing, training and using a customized software-based model and/or enhancing a production process, comprising a computer providing a software based Model Builder Module, an ontology database connected to the computer, input and output means connected to the computer for entering and outputting data by the computer, wherein the system is configured to perform the method steps of claims 1 to 11.

13. Computer program product comprising instructions which cause the involved computers to perform the method steps of claims 1 to 11.

14. Computer-readable storage medium and/or data carrier signal having stored thereon the computer program product of claim 13 which cause the involved computers to carry out the method steps of claims 1 to 11.
